# EUROPEAN PATENT APPLICATION

(11) **EP 0 663 211 A1**
(43) Date of publication of application: **19.07.1995**
(21) Application number: 94910029.1
(22) Date of filing: 16.03.1994
(51) Int. Cl.: A61K 38/00

(54) **AGENT FOR PREVENTING AND TREATING DIGESTIVE MUCOSAL DISORDERS**

(30) Priority: 17.03.1993 JP 83966/93
(71) Applicant: OTSUKA PHARMACEUTICAL CO., LTD., Chiyoda-ku Tokyo 101 (JP)
(72) Inventor: KISHI, Kyoichi, Tokushima 779-31 (JP); ROKUTAN, Kazuhito, Tokushima 770 (JP); KIDO, Yasuhiro, Tokushima 770 (JP); MUKAI, Kiyoshi 1-9, Aza Marusu Hiroshima, Itano-gun Tokushima 771-02 (JP); TAKAHASHI, Masayuki, Naruto-shi Tokushima 772 (JP)
(74) Representative: Hansen, Bernd, Dr. Dipl.-Chem.
(86) International application number: JP9400428
(87) International publication number: WO9421276

(57) **Abstract**

An agent for preventing and treating digestive mucosal disorders which comprises M-CSF as the active ingredient. By using this novel agent, digestive mucosal disorders caused by various tissue damage factors can be effectively treated.

## Description

### TECHNICAL FIELD

This invention relates to the medicinal use of M-CSF (macrophage colony stimulating factor) and more particularly to a pharmaceutical composition comprising said M-CSF as an active ingredient for the prophylaxis and therapy of gastrointestinal mucosal disorder.

### BACKGROUND ART

It is known that the gastrointestinal mucosal cells feature a high metabolic turnover and are highly susceptible to a variety of tissue-impairing agents. As typical tissue-impairing agents, the side effects of drugs can be mentioned. For example, the onset of gastrointestinal mucosal disorder following administration of anticancer agents and antibiotics has been reported for years. This is true with antiinflammatory drugs such as indomethacin and aspirin and a variety of gastrointestinal symptoms are ascribed to adverse reactions due to these drugs.

With regard to the gastrointestinal mucosal disorder mentioned above, not only the role played by such causative tissue-impairing agents but also effective countermeasures are being explored but what is done at most today is an attempt to dispose of the causative tissue-impairing agents as much as possible. Meanwhile, as a positive approach to the prevention and treatment of such gastrointestinal mucosal disorder, a glutamine-containing infusion has been developed and its efficacy is gathering attention and under scrutiny (JJPEN, vol. 14, No. 6, pp. 933-940, 1992).

Meanwhile, M-CSF is known to be a physiological factor whose biological activity is to stimulate the differentiation and proliferation of macrophage/monocyte lineage cells and is reportedly of use as, inter alia, an agent for the prophylaxis and therapy of the diseases or morbid states accompanied by leukocytopenia, an adjunct to surgery for bone marrow transplantation, a prophylactic and therapeutic agent for various infectious diseases, and even a therapeutic agent for certain types of cancer (JP Kokai H-2-2391). Furthermore, M-CSF has been demonstrated to have antiinflammatory and antiallergic actions and its usefulness as an antiinflammatory drug or an antiallergic drug has been reported (WO91/08754).

The object of this invention is to develop new medicinal uses for M-CSF which have not heretofore been known, particularly the use of M-CSF for the prevention and treatment of said gastrointestinal mucosal disorder.

### DISCLOSURE OF INVENTION

The inventors of this invention explored various prophylactic and therapeutic modalities for gastrointestinal mucosal disorder, and discovered surprisingly that quite beyond anticipation by analogy to its hitherto-reported pharmacologic actions, M-CSF produces satisfactory responses in said gastrointestinal mucosal disorder. This invention has been developed on the basis of the above finding.

In accordance with this invention there is provided a pharmaceutical composition for the prophylaxis and therapy of gastrointestinal mucosal disorder characterized in that it comprises M-CSF as an active ingredient.

The pharmaceutical composition of this invention for the prophylaxis and therapy of gastrointestinal mucosal disorder (hereinafter referred to briefly as the 'present medicine'), when administered, exerts pharmacologic actions, typically protecting the mucosa from damages and improving the function of the gastrointestinal system.

The gastrointestinal mucosal disorder in which the present medicine can be indicated includes a variety of mucosal disorders of the gastrointestinal tract which are caused by various tissue-impairing agents. Of these disorders, disorders of the intestinal tract mucosa are preferred targets of the present medicine and, in such disorders, this medicine produces both preventive and therapeutic effects.

The tissue-impairing agent includes a comprehensive list of agents which induce disorders of the gastrointestinal mucosa. Typical are injurious substances such as the drugs mentioned above and ethanol, long-term total parenteral nutrition (TPN), postoperative dissimilation, oxidative stress and so on. Among these agents, TPN causes a disorder characterized by atrophy of the intestinal tract, and the mucosal disorder in which the present medicine can be indicated naturally encompasses such atrophy and hypofunction of the intestinal system. These tissue-impairing agents and the gastrointestinal mucosal disorders caused by them are well known in the art.

M-CSF, as the active ingredient of the present medicine, includes a number of physiologically active substances which have the intrinsic physiological action of M-CSF, that is to say the property to act on hematopoietic precursors in the bonemarrow to promote the differentiation and proliferation of monocytes and macrophages (e.g. Science, 236, 1229, 1987). Of course, such M-CSF is not limited to native species but includes recombinant species which can be obtained by genetic engineering techniques. Furthermore, it may be any derivative of M-CSF that has similar activity.

Therefore, in this specification, the term 'M-CSF' is used in a broad comprehensive sense covering such native, recombinant and derivatized substances. Examples of such M-CSF are those species of human origin which are disclosed in EP-A 261592 and 328061 and WO91/06567, among others. For convenience's sake, these species of M-CSF are herein designated in accordance with the expression rule based on the amino acid sequence of the 554 amino acid precursor described in the above respective patent literature, assuming its 33rd amino acid residue Glu as position-1. M-CSF for use in this invention is preferably chosen from among those species of human origin which are disclosed in the above patent literature. Preferred, among them, are M-CSF species having a primary amino sequence with N-terminus in the region between Glu¹ and Glu⁵, inclusive, and C-terminus in the region between Glu¹⁴⁵ and Pro²¹⁴, inclusive, and, for still better results, between Thr¹⁵³ and Pro²¹⁴, inclusive. The optimum M-CSF for use in the present medicine is an M-CSF having a primary amino acid sequence from Val³ or Ser⁴ to Thr¹⁵³.

The present medicine is generally provided in a dosage form comprising a pharmacologically effective amount of M-CSF and an ordinary pharmaceutically acceptable nontoxic carrier. The dosage form depends on the route of administration. Thus, a solution, a suspension or an emulsion is generally chosen with advantage. Such dosage forms are generally administered orally, intravenously, subcutaneously, intradermally, intramuscularly or otherwise. Processing into said respective dosage forms can be carried out by the established pharmaceutical procedures and generally the proportion of the active ingredient in the final dosage form can be judiciously in the range of about 0.001-100 mg protein.

Of course, the present medicine is not limited to the above-mentioned liquid form but can be provided in a variety of dosage forms suited for oral or parenteral administration. It may be a lyophilized product which can be extemporaneously reconstituted into a liquid dosage form using a suitable aqueous vehicle.

The dosage of the present medicine is selected according to the desired pharmacologic effect, the patient's age and sex, the severity of disorder, etc. and cannot be stated in general terms. However, for adults, the recommendable dosage of the active substance is usually about 0.001-50 mg protein/kg/day and this dose can be administered once a day or in several divided doses or administered by intravenous drip. Particularly, efficacy-wise, the present medicine is preferably provided in an extemporaneously reconstitutable form for intravenous drip injection which can be diluted into a suitable parenteral vehicle or an ordinary infusion.

If desired, the present medicine may contain other pharmacologically active ingredients. For example, glutamines such as alanylglutamine are effective additional ingredients.

### BRIEF DESCRIPTION OF THE DRAWINGS

Fig. 1 is a photomicrogram (x 40 magnification) of an H & E-stained tissue section from the control infusion group (a photograph in lieu of the morphological drawing of a living matter).

Fig. 2 is a photomicrogram (x 40 magnification) of an H & E-stained tissue section from the M-CSF infusion group (100 µg/kg) (a photograph in lieu of the morphological drawing of a living matter).

Fig. 3 is a photomicrogram (x 40 magnification) of an H & E-stained tissue section from the M-CSF infusion group (250 µg/kg) (a photograph in lieu of the morphological drawing of a living matter).

Fig. 4 is a photomicrogram (x 100 magnification) of an H & E-stained tissue section from the control infusion group (a photograph in lieu of the morphological drawing of a living matter).

Fig. 5 is a photomicrogram (x 100 magnification) of an H & E-stained tissue section from the M-CSF infusion group (100 µg/kg) (a photograph in lieu of the morphological drawing of a living matter).

Fig. 6 is a photomicrogram (x 100 magnification) of an H & E-stained tissue section from the M-CSF infusion group (250 µg/kg) (a photograph in lieu of the morphological drawing of a living matter).

Fig. 7 is a photomicrogram (x 400 magnification) of a PCNA-stained tissue section from the control infusion group (a photograph in lieu of the morphological drawing of a living matter).

Fig. 8 is a photomicrogram (x 400 magnification) of a PCNA-stained tissue section from the M-CSF infusion group (250 µg/kg) (a photograph in lieu of the morphological drawing of a living matter).

Fig. 9 is a diagrammatic representation of the stimulating effects of epidermal growth factor (EGF), M-CSF and insulin on the proliferation of IEC-6 cells as determined in Test Example 2 which appears herein after.

Fig. 10 is a diagrammatic representation of the stimulating effects of EGF, basic fibroblast growth factor (bFGF), M-CSF, insulin, heparin and IL-1β on the proliferation of IEC-6 cells as determined in Test Example 2 which appears hereinafter.

Fig. 11 is a diagrammatic representation demonstrating that the promoting effect of M-CSF on the proliferation of IEC-6 cells is antagonized by its neutralizing antibody, with the corresponding effect of EGF being taken as control, which was verified in Test Example 2 which appears hereinafter.

### BEST MODE FOR PRACTICING THE INVENTION

The following test examples are intended to describe this invention in further detail.

### Test Example 1

As M-CSF, an M-CSF (an active substance comprising a polypeptide having an amino acid sequence from Ser⁴ to Thr¹⁵³) prepared using Escherichia coli in accordance with the method described in WO91/06567 was used.

The effect of M-CSF on gastrointestinal tract disorder was investigated using methotrexate (MTX)-lesioned rats in accordance with the method of Kido et al [JJPEN, 14(6), 933-940 (1992)].

Thus, male Wistar rats (Japan SLC, Std Wistar/ST) weighing about 200 g were operated on for indwelling venous catheterization and an MTX-containing infusion of the following composition was administered to construct models of gastrointestinal tract disorder (control group) (Dosage of MTX: 0.3 mg/kg/day).

### 〈Infusion formula〉

| | |
|---|---|
| GE-3 (Basic Pharmacology and Therapeutics, 20, Supplemental Issue 2, pp. 645-655, 1992) | 140 ml |
| Amiparen (registered TM, Otsuka Pharmaceutical) | 89.2 ml |
| Otsuka MV Injection (Otsuka Pharmaceutical) | 0.1 ml |
| MTX (Takeda Chemical Industries) | 0.3 ml |
| Distilled water (Otsuka Pharmaceutical) | to make 270 ml |

As test groups, groups given infusions prepared by adding 100 µg and 250 µg, respectively, of said M-CSF to portions of the above infusion (M-CSF infusion groups; n=6 for each concentration of M-CSF) were provided. In each of the M-CSF infusion groups and the M-CSF-free control infusion group (control infusion group, n=6), histological examination was performed to evaluate the efficacy of administered M-CSF.

The histological examination was carried out on tissue sections prepared in the routine manner and subjected to hematoxylin-eosin (H & E) staining and immunostaining with anti-PCNA (proliferating cell nuclear antigen) antibody.

The H & E staining mentioned above was carried out as follows. In the routine manner, the tissue section was deparaffinized, washed in running water and nuclearly stained with hematoxylin. After the free dye was washed off in running water, the cytoplasm was stained with eosin. Then, the specimen was dehydrated in an alcohol series, cleared with xylene, and sealed with marinol.

The immunostaining with anti-PCNA antibody was carried out as follows. First, the tissue section was deparaffinized and immersed in 0.3% H₂O₂-methanol (1.5 ml of 30% H₂O₂ added to 150 ml of methanol) (prepared extemporaneously) at room temperature for 30 minutes to inactivate the endogenous peroxidase. The tissue specimen was then treated with alcohol, transferred into water and saturated with PBS. To this section was added a blocking reagent (10% normal bovine serum, Seikagaku Corporation) and after 30 minutes' immersion at room temperature, the excess moisture was removed. Then, without washing the specimen, anti-human PCNA monoclonal mouse antibody (DAKO-PCNA, PC10, Dako) was added as a primary antibody. The specimen was kept immersed at room temperature for 1 hour.

As negative control, the blocking reagent was used in lieu of the primary antibody. Then, the specimen was washed with PBS and a secondary antibody (biotin-labeled anti-mouse IgG + IgA + IgM antibody, Seikagaku Corporation) was added. The specimen was kept immersed at room temperature for 30 minutes. The specimen was then washed with PBS and immersed in DAB-H₂O₂ solution (prepared from 30 mg of 3,3'-diaminobenzidine-4HCl (Wako Pure Chemical), 135 ml of distilled water, 15 ml of 0.5 M Tris-HCl buffer (pH 7.6) and 0.3 ml of 2.5% H₂O₂-water immediately before use) for about 5-10 minutes for color development. As the blocking reagent, secondary antibody and standard reagent, the components of Histofine Stain Kit (Seikagaku Corporation) were used.

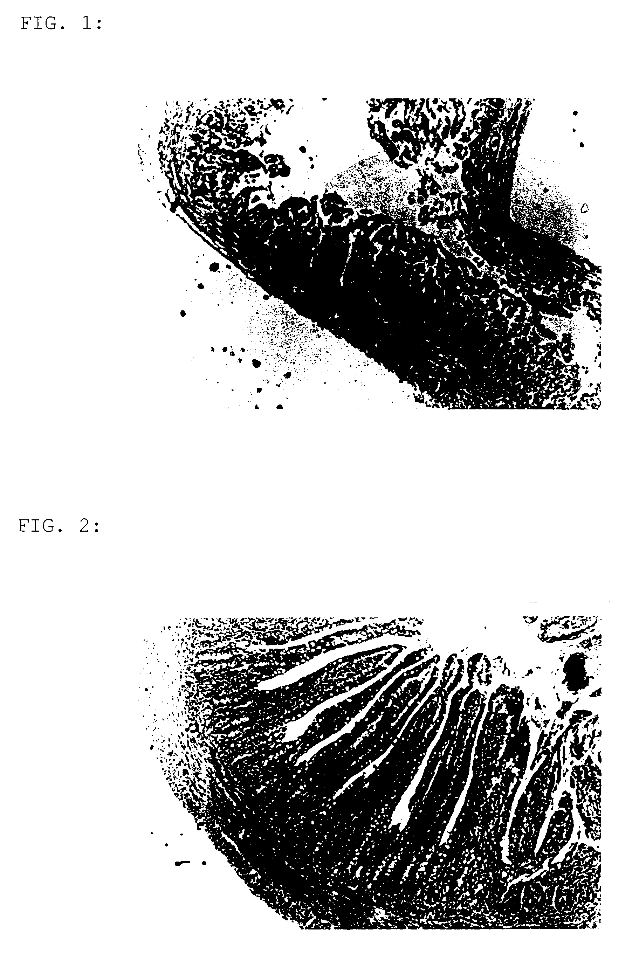

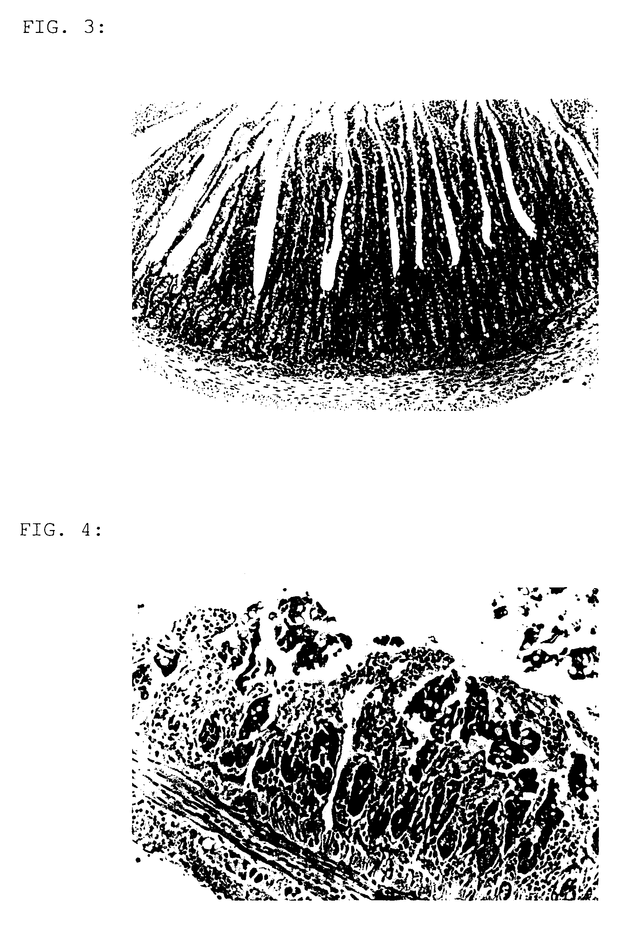

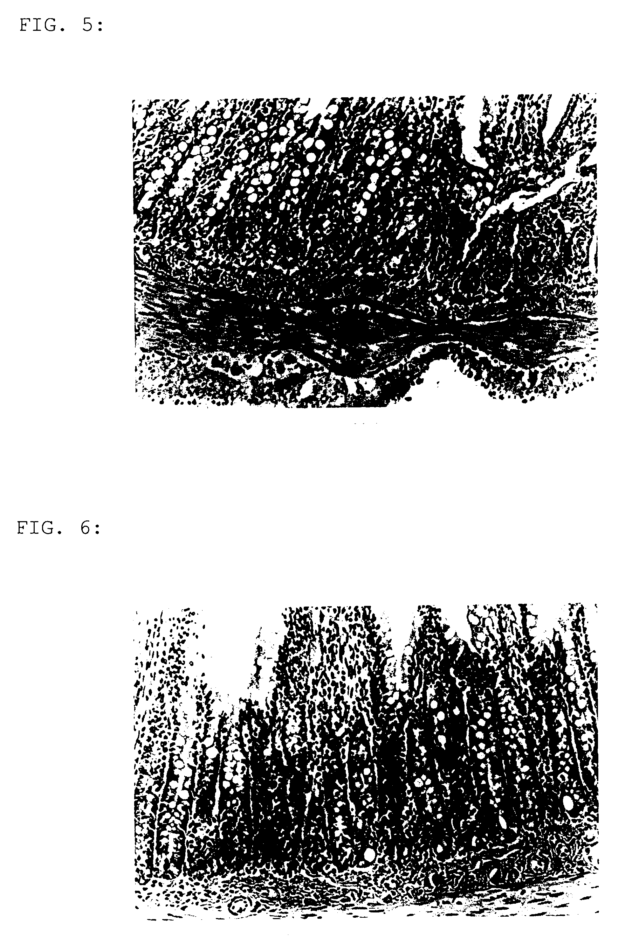

Results of the above test are shown in Figs. 1 through 8.

The following is apparent from the drawings. As MTX-induced injury of small intestinal epithelial cells, it is known that this substance causes swelling and cytoplasmic vacuolation of the mucosal cells, desquamation of the epithelial cells, leakage of plasma into the intestinal lumen, leukocyte infiltration into the submucosa, and ultimate onset of serious desquamative enteritis affecting the entire course of the intestinal canal. In the present test, too, 7-day administration of the MTX-containing infusion (control group) caused serious desquamative jejunitis (Figs. 1 and 4).

However, when 100 µg or 250 µg of M-CSF was added to the same MTX-containing infusion fluid and the resulting composition administered for 7 days (M-CSF infusion groups), the jejunal villi and crypt architecture were well retained in all cases, indicating that the MTX-associated desquamative enteritis was effectively controlled (Figs. 2, 3, 5 and 6). The above effect of M-CSF on the jejunum was paralleled in the duodenum and ileum as well.

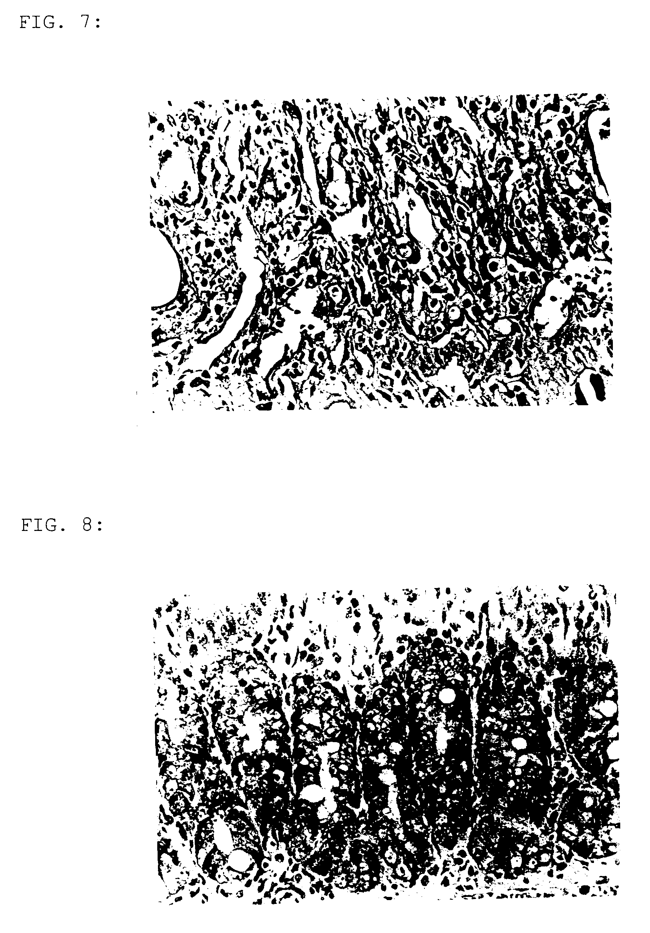

Furthermore, whereas substantially no PCNA-positive cells were found in the control infusion group, PCNA-positive cells were found among the crypt cells in the M-CSF infusion groups (Figs. 7 and 8).

### Test Example 2

This test was performed to see whether, in the rat model of small intestinal mucosal lesion induced by administration of MTX to rats, M-CSF would show a mucosa-protecting action.

As the mechanism of this small intestinal mucosa-protecting action of M-CSF, the stimulating effect of M-CSF on the proliferation of small intestinal epithelial cells may be postulated. Thus, the small intestinal epithelial cell is derived from the undifferentiated cell in the crypt and as this crypt cell undergoes proliferative mitosis and migrates toward the intestinal lumen, it becomes differentiated into functional, small intestinal epithelial cells. Then, the cell sheds off from the tip of the villus. Therefore, the maintenance of the small intestinal mucosa is largely dependent on the proliferation of crypt cells. In order to demonstrate that the small intestinal mucosa-protecting action of M-CSF is derived from its stimulating effect on the proliferation of small intestinal crypt cells, the following in vitro test using IEC-6 cells, which are a small intestinal epithelial cell line derived from rat small intestinal crypt cells, was performed.

### (1) Method

IEC-6 cells were purchased from ATCC and maintained by serial passage through DMEM medium supplemented with 5% fetal calf serum (FCS), 0.1 U/ml insulin, 0.1 mg/ml streptomycin and 100 U/ml penicillin G. In the experiment, cells of the 20-25th generation were used.

In the cell proliferation experiment using a 24-well microtiter plate, 4x10⁴ cells per well were incubated in low-serum medium (the FCS concentration of the above-mentioned culture medium was reduced to 0.1%) for 12 hours. The grown cells were exfoliated with trypsin-EDTA solution and counted in a hemocytometer and the result was regarded as the cell count on day 0. Then, the M-CSF mentioned above, epidermal growth factor (EGF: mouse, receptor grade; Becton-Dickinson), insulin (bovine pancreatic origin; Sigma), basic fibroblast growth factor (bFGF: human; Oncogene Science) and interleukin 1β (IL-1β; Kikumoto et al., Biochem. Biophys. Res. Commun. 147, 315-321, 1987) were respectively added at various final concentrations and the number of cells was determined on day 2 and day 5 in the same manner as above. Furthermore, the above experiment was repeated by adding the M-CSF neutralizing antibody (monoclonal antibody ANOC573 described in JP Kokai H-5-95794: 1.2 mg/ml) at various concentrations.

### (2) Results

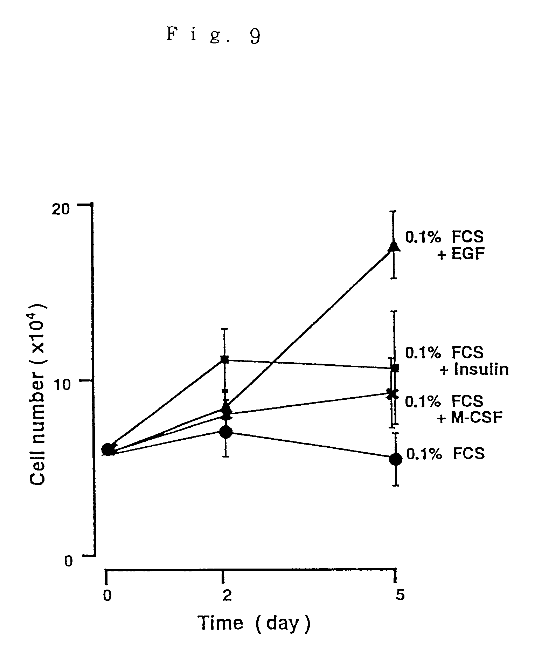

Fig. 9 shows a comparison of proliferation-stimulating effect among EGF which shows the most potent action (0.1 µg/ml), insulin which is a representative growth factor (0.1 IU/ml) and M-CSF (1 µg/ml) using the number of cells cultured in 0.1% FCS-containing DMEM as control. Each data is mean ±SD (n=9).

It is apparent from Fig. 9 that M-CSF is approximately 1/2 as potent as EGF and roughly equipotent to insulin.

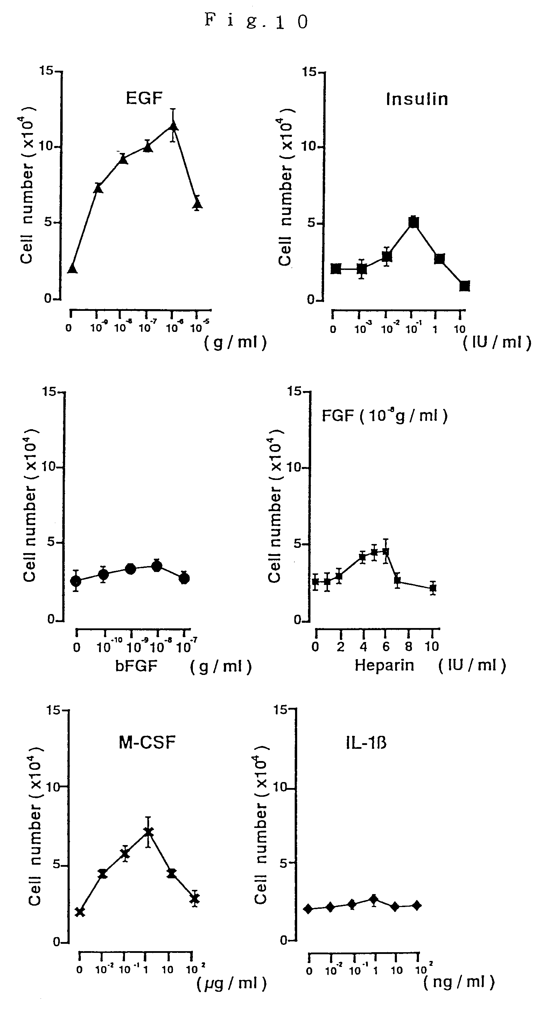

In addition to the effect of these growth factors, the growth-stimulating effect of bFGF and IL-1β which are released from M-CSF-activated macrophages were also investigated. Thus, in the same manner as described above, EGF, insulin, M-CSF, bFGF and IL-1β were respectively added at various concentrations and the number of cells on day 5 was counted. With regard to bFGF, using its optimum concentration (0.01 µg/ml), heparin having an bFGF-stabilizing action was added and the number of cells was counted to investigate its growth-promoting effect. The data [mean ± SD (n=9)] are presented in Fig. 10.

It is clear from Fig. 10 that M-CSF is approximately 1/2 as potent as EGF and that its optimum concentration is 1 µg/ml. It is also apparent from the graph that M-CSF is equipotent to, or more potent than, insulin and bFGF in the promotion of cell proliferation. No such proliferation-promoting effect was found with IL-1β.

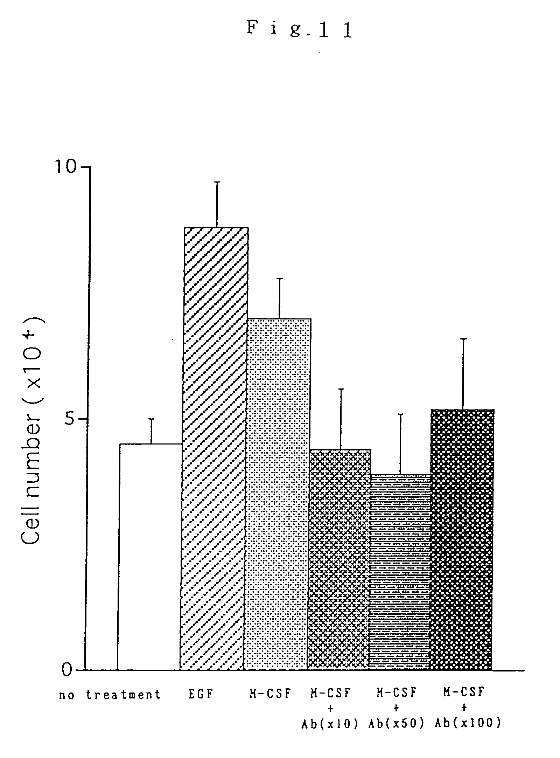

Then, the above cell proliferation-promoting action of M-CSF was verified by an antagonizing experiment using a neutralizing antibody. To the cells in the 0.1 µg/ml M-CSF group, 10-, 50- and 100-fold dilutions (final) of the antibody were added and the number of cells was determined on day 5 in the same manner as described above. As positive control, 1 µg/ml of EGF was added and the number of cells was determined. The results (mean ± SD, n=9) are shown in Fig. 11.

It is apparent from Fig. 11 that the stimulating effect of M-CSF on the proliferation of IEC-6 cells was completely inhibited by the presence of the neutralizing antibody (concentrations not less than 50-fold dilution).

Thus, in this in vitro experiment, the small intestinal mucosa-protecting effect of M-CSF as observed in vivo was confirmed to be ascribable to the direct growth-stimulating action of M-CSF on the small intestinal crypt cells. It was also confirmed that when the proliferation of cells treated with EGF was used as positive control, M-CSF was consistently about 1/2 as potent. These findings indicated that M-CSF promotes proliferation of epithelial cells and is effective in the protection of gastrointestinal mucosa against the injurious effects of various agents.

### INDUSTRIAL APPLICABILITY

In accordance with this invention, there is provided a novel pharmaceutical composition for the prophylaxis and therapy of gastrointestinal mucosal disorder, and gastrointestinal mucosal disorders arising from various tissue-impairing agents can be effectively controlled by this invention.

## Claims

1. A pharmaceutical composition for the prophylaxis and therapy of gastrointestinal mucosal disorder characterized in that said composition comprises a pharmacologically effective amount of M-CSF in combination with a pharmaceutically acceptable nontoxic carrier.

2. The pharmaceutical composition for the prophylaxis and therapy of gastrointestinal mucosal disorder as claimed in claim 1 wherein said M-CSF is an M-CSF having a primary amino acid sequence with N-terminus somewhere between Glu in 1-position and Glu in 5-position and C-terminus somewhere between Glu in 145-position and Pro in 214 position.

3. The pharmaceutical composition for the prophylaxis and therapy of gastrointestinal mucosal disorder as claimed in claim 1 wherein said M-CSF is an M-CSF having a primary amino acid sequence from Val in 3-position to Thr in 153 position.

4. The pharmaceutical composition for the prophylaxis and therapy of gastrointestinal mucosal disorder as claimed in claim 1 wherein said M-CSF is one having a primary amino acid sequence from Ser in 4-position to Thr in 153 position.

5. The pharmaceutical composition for the prophylaxis and therapy of gastrointestinal mucosal disorder as claimed in claim 1 which is a gastrointestinal mucosa-protecting and gastrointestinal function-improving drug.

6. The pharmaceutical composition for the prophylaxis and therapy of gastrointestinal mucosal disorder as claimed in claim 1, the pharmacologically effective dosage of which is 0.001-50 mg protein/kg/day.

7. A method for the prophylaxis and therapy of gastrointestinal mucosal disorder characterized in that a pharmacologically effective amount of M-CSF is administered to a patient requiring the prevention or therapy of gastrointestinal mucosal disorder.

8. Use of M-CSF for the production of a pharmaceutical composition for the prophylaxis and therapy of gastrointestinal mucosal disorder.

9. A method for producing a pharmaceutical composition for the prophylaxis and therapy of gastrointestinal mucosal disorder characterized by mixing a pharmacologically effective amount of M-CSF as an active ingredient with a pharmaceutically acceptable nontoxic carrier.
